# EUROPEAN PATENT APPLICATION

(11) **EP 4 542 215 A1**
(43) Date of publication of application: **23.04.2025**
(21) Application number: 23849375.3
(22) Date of filing: 31.07.2023
(51) Int. Cl.: G01N 27/401, G01N 27/416, G01N 27/26

(54) **SAMPLE TEST CARD**

(30) Priority: 31.07.2022 CN 202210912914
(71) Applicant: Edan Instruments, Inc., Shenzhen, Guangdong 518000 (CN)
(72) Inventor: LI, Guoxia, Shenzhen, Guangdong 518000 (CN); YAN, Bing, Shenzhen, Guangdong 518000 (CN); LIU, Xiaoying, Shenzhen, Guangdong 518000 (CN); ZHANG, Xing, Shenzhen, Guangdong 518000 (CN); SHI, Minglian, Shenzhen, Guangdong 518000 (CN)
(74) Representative: Herrero & Asociados, S.L.
(86) International application number: PCT/CN2023/110395
(87) International publication number: WO 2024/027668

(57) **Abstract**

A sample test card (9), relating to the field of analytical instruments. The sample test card (9) comprises a first channel (1), a second channel (2), a first electrode (41) located in the first channel (1), and a second electrode (42) located in the second channel (2). The first electrode (41) is configured to acquire electrochemical parameters of a sample in the first channel (1), and the second electrode (42) is configured to acquire electrochemical parameters of a reference liquid in the second channel (2). The sample test card (9) further comprises a salt bridge (3), one end of the salt bridge (3) is exposed in the first channel (1), and the other end of the salt bridge (3) is exposed in the second channel (2). The sample test card (9) can achieve the connection of the first electrode (41) and the second electrode (42) by using the salt bridge (3), thereby improving the accuracy of the measured electrochemical parameters of the sample.

## Description

### TECHNICAL FIELD

The present disclosure relates to the field of analytical instruments, and in particular to a sample test card.

### BACKGROUND

In order to detect a biochemical sample, a plurality of electrodes may be arranged in a sample test card to detect the sample by means of electrochemistry. Therefore, it is necessary to form a conductive circuit between the electrodes in the test card to test an electrochemical parameter of the sample.

At present, in this type of sample test card, there is usually a permeable membrane arranged between a reference cell with a reference electrode and a measurement channel with a measuring electrode, or a reference solution in the reference cell is in direct contact with the sample in the measurement channel, thereby establishing the conductive circuit. However, these methods may cause compositions of the reference solution and compositions of the sample to be doped with each other, which is not conducive to the stability of a measurement environment and thus affects the accuracy of measurement results. In addition, if the reference solution is in direct contact with the sample when testing, a liquid junction potential between the reference solution and the sample may also affect the accuracy.

### SUMMARY

Some embodiments of the present disclosure provide a sample test card to improve accuracy of measuring an electrochemical parameter of a sample.

Some embodiments of the present disclosure provide a sample test card including a first channel, a second channel, a first electrode arranged in the first channel, and a second electrode arranged in the second channel. The first electrode is configured to obtain an electrochemical parameter of a sample in the first channel. The second electrode is configured to obtain an electrochemical parameter of a reference solution in the second channel. The sample test card further includes a salt bridge. An end of the salt bridge is exposed in the first channel, and another end of the salt bridge is exposed in the second channel.

Different from the related art, some embodiments of the present disclosure provide the sample test card by arranging the salt bridge between the first channel filled with the sample and the second channel filled with the reference solution, enabling the first electrode in the first channel to be conductive with the second electrode in the second channel. In this way, a conductive circuit is established while liquids in the first channel and the second channel are not in direct contact with each other, thereby effectively avoiding mutual doping and contamination of the liquids. By taking advantage of a characteristic that a migration rate of cations in the salt bridge is different from a migration rate of anions in the salt bridge, influence of a liquid junction potential on measurement results of the electrochemical parameter is effectively reduced, thereby ensuring stability of the reference solution and the sample and improving accuracy of measuring the electrochemical parameter of the sample.

### BRIEF DESCRIPTION OF THE DRAWINGS

In order to illustrate the technical solutions in the embodiments of the present disclosure more clearly, the drawings that need to be used in the embodiments are briefly introduced below. Obviously, the drawings in the following description are merely some embodiments of the present disclosure. For those skilled in the art, other drawings may be obtained according to these drawings without making creative efforts.
FIG. 1 is a schematic assembled structural view of a sample test card according to some embodiments of the present disclosure.
FIG. 2 is a schematic exploded structural view of the sample test card shown in FIG. 1 according to some embodiments of the present disclosure.
FIG. 3 is a schematic assembled structural view of a first plate and a second plate shown in FIG. 1 according to some embodiments of the present disclosure.
FIG. 4 is a schematic structural view of the first plate shown in FIG. 1 according to some embodiments of the present disclosure.
FIG. 5 is a schematic assembled structural view of the first plate and the second plate shown in FIG. 1 from another view according to some embodiments of the present disclosure.
FIG. 6 is a schematic enlarged structural view of a region A shown in FIG. 5 according to some embodiments of the present disclosure.
FIG. 7 is a schematic structural view of the sample test card shown in FIG. 1 from another view according to some embodiments of the present disclosure.
FIG. 8 is a schematic cross-sectional view along a C-C direction shown in FIG. 3 according to some embodiments of the present disclosure.
FIG. 9 is a schematic enlarged structural view of a region B shown in FIG. 8 according to some embodiments of the present disclosure.

### DETAILED DESCRIPTION

In order to make the purposes, features, and effects of the present disclosure mentioned above more obvious and easy to understand, implementations of the present disclosure are described in detail below with reference to accompanying drawings. It can be understood that the implementations described here are merely used to explain the present disclosure, but not to limit the present disclosure. In addition, it should be noted that, for convenience of description, only some but not all structures related to the present disclosure are shown in the drawings. Based on the embodiments of the present disclosure, all other embodiments obtained by those of ordinary skill in the art without creative efforts fall within a scope of protection of the present disclosure.

The terms "first", "second", etc. in the present disclosure are used to distinguish different objects, rather than describing a specific sequence. In addition, the terms "include" and "arranged with" as well as any variations of thereof are intended to cover non-exclusive inclusions. For example, processes, methods, systems, products, or devices that include a series of steps or units are not limited to the steps or units listed, but may further include steps or units that are not listed, or may still include other steps or units that are inherent to the processes, methods, products, or devices.

The term "embodiment" in the present disclosure means that a specific feature, structure, or characteristic described in conjunction with the embodiments may be included in at least one embodiment of the present disclosure. Appearances of the term in various positions in the description do not necessarily refer to a same embodiment, nor are they separate embodiments or alternative embodiments that are mutually exclusive with other embodiments. It is understood explicitly and implicitly by those skilled in the art that the embodiments described herein may be combined with other embodiments.

With development of instrumental analysis technologies, a biochemical sample is usually tested by means of a test card, so as to improve testing efficiency and keep the sample from being contaminated, thereby ensuring accuracy of the test. The sample test card may detect the sample by means of measuring an electrochemical parameter. In a test card configured for a blood gas analyzer, a three-electrode system including a reference electrode, an ion electrode, and a counter electrode that form a circuit may be used to test the sample.

In the related art, a permeable membrane may be arranged between a reference cell and a measurement channel, or a reference solution in the reference cell may be in direct contact with a liquid in the measurement channel, enabling the reference cell arranged with the reference electrode to be conductive with the measurement channel arranged with the ion electrode and the counter electrode.

During a measurement process, at a position where the reference cell is in contact with and conductive with the measurement channel, that is, when the reference solution in the reference cell is conductive with the liquid in the measurement channel, and two electrolytes with different compositions or activities are in contact with each other, at a junction of solutions of the two electrolytes, since a migration speed of positive ions is different from a migration speed of negative ions when the ions across the junction, positive charges and negative charges may separate from each other and form an electrical double layer. A potential difference generated in the electrical double layer is called a liquid junction diffusion potential, or a liquid junction potential for short. Since the liquid junction potential may affect measurement results, a liquid junction potential and an impedance between the reference cell and the measurement channel need to be as small as possible during the measurement process. In addition, the electrodes need to be disposed in a stable environment when measuring the electrochemical parameter, and the sample to be tested should not be as uncontaminated as possible. Therefore, a volume, a concentration, and material composition of the reference solution and a volume, a concentration, and material composition of the liquid in the measurement channel need to be kept stable. Thus, when an electrode in the reference cell is conductive with an electrode in the measurement channel, it is necessary that the liquid in the measurement channel and the liquid in the reference cell cannot be doped and contaminated with each other.

However, in the related art, the conduction between the electrodes may be achieved by means of arranging the permeable membrane between the reference cell and the measurement channel, or by the direct contact of the reference solution in the reference cell with the liquid in the measurement channel, however, neither of these methods may satisfactorily meet requirements mentioned above and may have adverse impact on the accuracy of detection of the electrochemical parameter.

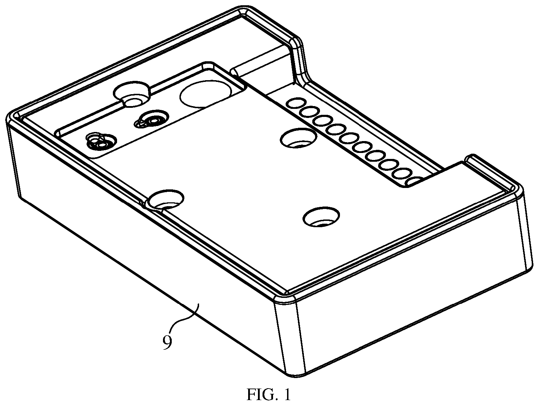

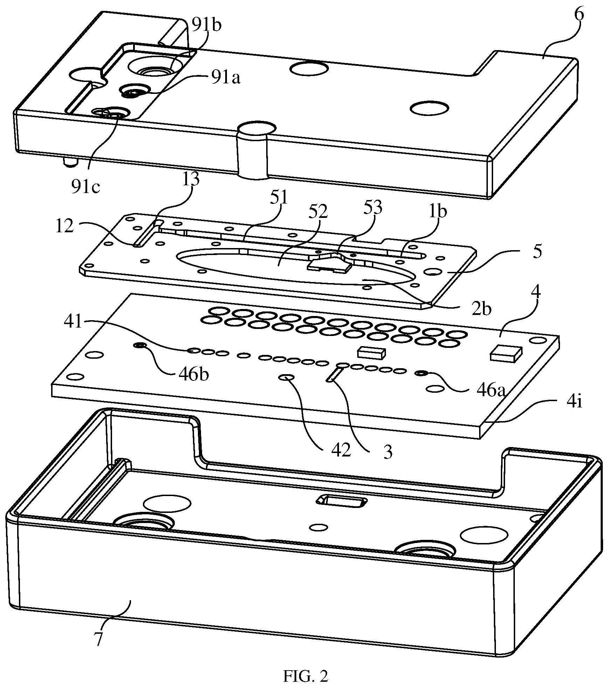

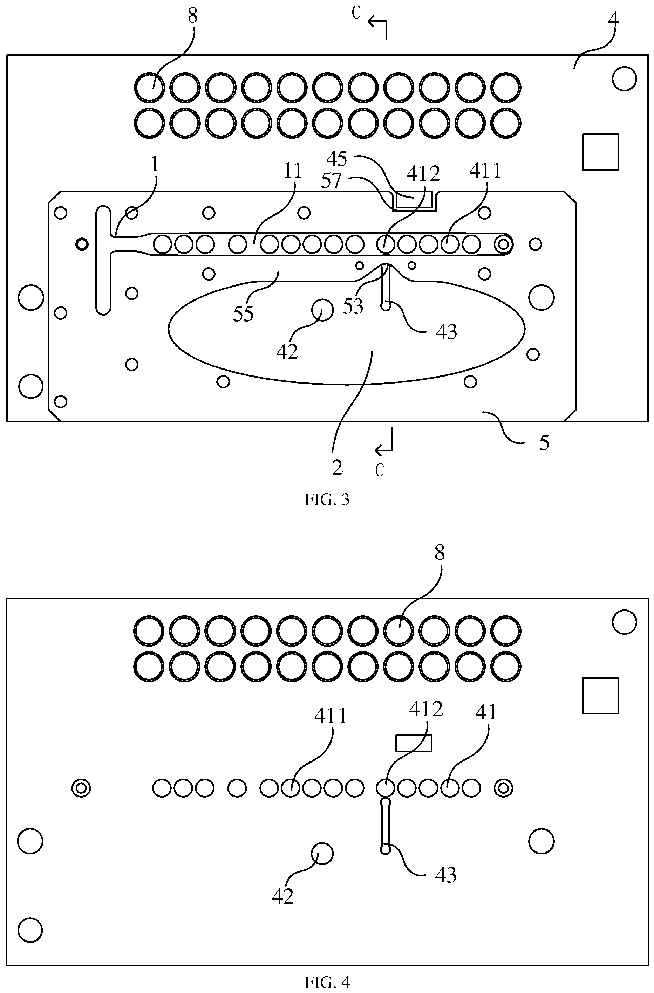

In order to ensure the accuracy of sample detection, some embodiments of the present disclosure provide a sample test card 9. As shown in FIG. 1 to FIG. 3, FIG. 1 is a schematic assembled structural view of a sample test card according to some embodiments of the present disclosure, FIG. 2 is a schematic exploded structural view of the sample test card shown in FIG. 1 according to some embodiments of the present disclosure, and FIG. 3 is a schematic assembled structural view of a first plate and a second plate shown in FIG. 1 according to some embodiments of the present disclosure. Some embodiments of the present disclosure provide the sample test card 9. The sample test card 9 includes a first channel 1, a second channel 2, a first electrode 41 located in the first channel 1, and a second electrode 42 located in the second channel 2. The first electrode 41 is configured to obtain an electrochemical parameter of a sample in the first channel 1. The second electrode 42 is configured to obtain an electrochemical parameter of a reference solution in the second channel 2. In order to enable the first electrode 41 to be conductive with the second electrode 42, the sample test card 9 further includes a salt bridge 3. An end of the salt bridge 3 is exposed to/in the first channel 1, and the other end of the salt bridge 3 is exposed to/in the second channel 2.

In above embodiments, the salt bridge 3 may be arranged between the first channel 1 receiving the sample and the second channel 2 receiving the reference solution, enabling the first electrode 41 in the first channel 1 to be conductive with the second electrode 42 in the second channel. In this way, a conductive circuit is established while liquids in the first channel 1 and the second channel 2 are not in direct contact with each other, thereby effectively avoiding mutual doping and contamination of the liquids. Due to a characteristic that a migration rate of cations in the salt bridge 3 is different from a migration rate of anions in the salt bridge 3, influence of a liquid junction potential on measurements results of the electrochemical parameter is effectively reduced, thereby ensuring stability of the reference solution and the sample and improving accuracy of measuring the electrochemical parameter of the sample.

In some embodiments, since ions in the salt bridge 3 are continuously migrating in a directional manner, an ion concentration near the salt bridge 3 changes significantly. When an electrode is too close to the salt bridge 3, it is not conducive for the electrode to measure the electrochemical parameter stably. Therefore, although the salt bridge 3 is close to the first electrode 41 and the second electrode 42, the salt bridge 3 still needs to keep a certain distance from both the first electrode 41 and the second electrode 42 for buffering, enabling the first electrode 41 and the second electrode 42 to perform testing in an environment with a relatively stable concentration.

In some embodiments, a large amount of liquid is usually required in the second channel 2 to provide a stable testing environment for performing a test on the sample, and a circuit structure arranged in the first channel 1 for testing the sample may be more complex. In order to leave sufficient space for accommodating the first electrode 41 and the circuit structure in the first channel 1, and a space of the second channel 2 is generally larger than a space of the first channel 1, a distance between the salt bridge 3 and the second electrode 42 in the second channel 2 is larger than a distance between the salt bridge 3 and the first electrode 41 in the first channel 1.

In some embodiments, the sample test card 9 may also include a second housing 7, thereby realizing an overall assembly and fixation of the sample test card 9. The second housing 7 presses against a first plate 4 at a side of the first plate 4 away from a second plate 5, enabling the first plate 4, the second plate 5, and a first housing 6 to be tightly fixed with each other, thereby avoiding situations such as inaccurate measurement results or damage to instruments and devices caused by leakage of liquids in the first channel 1 and the second channel 2.

In some embodiments, in a three-electrode system, the first electrode 41 arranged in a measurement channel 11 of the first channel 1 may include a counter electrode 412 and an ion electrode 411. The second electrode 42 arranged in the second channel 2 may include a reference electrode. In this way, the counter electrode 412, the ion electrode 411, and the reference electrode are conductive with each other via the salt bridge 3 and form the three-electrode system for testing the sample.

The counter electrode 412 is configured to form a circuit with the ion electrode 411, ensuring smooth flow of current through the ion electrode 411, enabling a reaction for testing the sample to occur on the ion electrode 411, thus the sample in the first channel 1 may be tested. The reference electrode is configured to form a circuit with the ion electrode 411 through the salt bridge 3, thereby providing a reference electrochemical parameter in the reference solution of the second channel 2 to determine an electrochemical parameter at the ion electrode 411.

In some embodiments, as shown in FIG. 3, the counter electrode 412 may be arranged at a position in the measurement channel 11 closest to the salt bridge 3. A plurality of ion electrodes 411 may be arranged along the measurement channel 11.

In some embodiments, since ions migrating from the salt bridge 3 are difficult to be evenly distributed rapidly in the channel, a change of ion concentration in a local area close to the salt bridge 3 may lead to inaccuracies in measurement results of the electrode. Therefore, the second electrode 42 in the second channel 2 may be located at a position close to the salt bridge 3 but still maintains a certain distance from the salt bridge 3.

In some embodiments, during an actual measurement process, a liquid in the measurement channel 11 may need to be replaced. For example, a calibration solution, a cleaning solution, etc. is introduced into the measurement channel 11 to complete measurement preparation before introducing the sample. After the liquid is drained, it may be difficult to conduct a circuit between the first electrode 41 and the second electrode 42 due to a lack of a medium. The circuit may be turned on and off many times when replacing the liquid, which is not conducive to stability of an electrochemical measurement system, and may have adverse impact on the measurement results. Therefore, the counter electrode 412 in the measuring channel 11 needs to be arranged in a position very close to the salt bridge 3. In this way, when the liquid is drained from the measurement channel 11, a liquid film that conducts the salt bridge 3 and the first electrode 41 may be formed by a small amount of remaining liquid to maintain conduction of a circuit of the three-electrode system.

In some embodiments, in order to avoid a circuit of the first plate 4 from malfunctioning due to liquid leakage, the first electrode 41 in the first channel 1 is not in direct contact with the salt bridge 3. A coating may be arranged on a surface of the first plate 4 at a region corresponding to the first channel 1 and a surface of the first plate 4 at a region corresponding to the second channel 2, so as to better protect the circuit structure on the first plate 4.

In some embodiments, the sample test card 9 may further be arranged with a plurality of third electrodes 8. Each of the third electrodes 8 is connected to a corresponding one of the first electrode 41 and the second electrode 42, enabling the third electrodes 8 to obtain the electrochemical parameters measured by the first electrode 41 and the second electrode 42, and to output the electrochemical parameters to a medical testing device (not shown) assembled with the sample test card 9. The medical testing device may analyze a composition and other properties of the sample based on the electrochemical parameters to obtain the measurement results of the sample.

In some embodiments, the sample that could be tested by the sample test card 9 may be a biochemical sample collected from an organism. In order to ensure that the sample does not deteriorate as much as possible during testing, a temperature measuring assembly 45 may be arranged on the first plate 4 to measure a temperature in the sample test card 9.

In some embodiments, the temperature measuring assembly 45 may be a thermistor protruding from a side of the first plate 4 towards the first channel 1. A second depression 57 may be defined on the second plate 5 at a position corresponding to the temperature measuring assembly 45 so as to make way or provide avoidance for the thermistor and ensure sealing performance of the sample test card 9. As shown in FIG. 3, the temperature measuring assembly 45 may be arranged at a position close to an edge of the second plate 5, and the second depression 57 may directly be recessed from the edge of the second plate 5 towards an interior of the second plate 5, so as to facilitate molding and processing of the second plate 5.

In some embodiments, as shown in FIG. 1 and FIG. 2, the first plate 4 may include a circuit board 4i. The circuit board 4i is arranged on a side of the first plate 4 close to the first channel 1 and the second channel 2. The salt bridge 3, the first electrode 41, and the second electrode 42 may be arranged on a surface of a side of the circuit board 4i towards the first channel 1, thereby contacting the sample in the first channel 1 and the reference solution in the second channel 2.

In some embodiments, the first plate 4 may also define a liquid inlet 46a and a liquid outlet 46b, which are configured to introduce the sample and the reference solution into the sample test card 9. The sample test card 9 further includes a first valve 91a, a second valve 91b, and a third valve 91c. The liquid inlet 46a is communicated with an end of the first channel 1. The other end of the first channel 1 includes a first branch 12 and a second branch 13. The first branch 12 is communicated with the liquid outlet 46b through the first valve 91a. The second branch 13 is communicated with an end of the second channel 2 through the second valve 91b. The other end of the second channel 2 is communicated with the liquid outlet 46b through the third valve 91c.

As shown in FIG. 2 to FIG. 4, FIG. 4 is a schematic structural view of the first plate shown in FIG. 1 according to some embodiments of the present disclosure. It can be found that due to structural limitations of the first channel 1 and the second channel 2 in the sample test card in the embodiments, and in order to facilitate an arrangement of the first electrode 41, a surface area of a portion of the salt bridge 3 exposed in the first channel 1 may be smaller than a surface area of another portion of the salt bridge 3 exposed in the second channel 2. In some embodiments, a length of the portion of the salt bridge 3 exposed in the first channel 1 may be smaller than a length of the portion of the salt bridge 3 exposed in the second channel 2. In this way, the salt bridge 3 may make way for the electrode while conducting the circuit, so as to achieve a good measurement effect.

A large amount of liquid is usually required in the second channel 2 to provide a stable testing environment for facilitating the testing of the sample. The circuit structure arranged in the first channel 1 for testing the sample may be complex. In order to leave sufficient space for accommodating the first electrode 41 and the circuit structure in the first channel 1, and a space reserved for placing the salt bridge in the second channel 2 is generally larger than a space reserved for the salt bridge in the first channel 1, the length of the another portion of the salt bridge 3 arranged in the second channel 2 may be larger than the length of the portion of the salt bridge 3 arranged in the first channel 1. Alternatively, the surface area of the another portion of the salt bridge 3 arranged in the second channel 2 may be larger than the surface area of the portion of the salt bridge 3 arranged in the first channel 1.

In some embodiments, for the convenience of arranging the salt bridge 3 and the stability of the measurement system, the salt bridge 3 may be a solid salt bridge made of hydrogel containing electrolytes.

In some embodiments, in addition to hydrogel, a material of the salt bridge 3 may also be agar, silica gel, gelatinized starch, or other materials.

In some embodiments, an impedance of the salt bridge may range from 1 kΩ to 1000 kΩ.

In some embodiments, compositions of the electrolyte contained in the salt bridge 3, the liquid introduced into the first channel 1, and the liquid introduced into the second channel 2 may match each other. That is to say, the electrolyte of the salt bridge 3 may hardly react with the liquid in the first channel 1 and the liquid in the second channel 2, and the electrolyte of the salt bridge 3, the liquid in the first channel 1, and the liquid in the second channel 2 may hardly contaminate each other, thereby achieving a better measurement effect.

In some embodiments, the first channel 1 and the second channel 2 may be electronically connected with each other through a third channel (not shown). The third channel is filled with the salt bridge 3. The salt bridge 3 is capable of expanding after contacting with a liquid introduced into the sample test card 9, thereby blocking the third channel to avoid the sample in the first channel 1 from being in direct contact with the reference solution in the second channel 2.

In some embodiments, the sample test card 9 may also include the first plate 4. A surface of the first plate 4 may define a groove 43 for filling the salt bridge 3. The first electrode 41 and the second electrode 42 are arranged on a surface of a side of the first plate 4 defining the groove 43. An end of the salt bridge 3 filled in the groove 43 is close to the first electrode 41, and the other end of the salt bridge 3 is close to the second electrode 42. In this way, the conduction of the circuit may be realized when the liquids are filled in the first channel 1 and the second channel 2.

In some embodiments, the sample test card 9 may also include the second plate 5. The second plate 5 defines a first through opening 51 and a second through opening 52. The second plate 5 is arranged on a side of the first plate 4 arranged with the groove 43. The end of the salt bridge 3 and the first electrode 41 are exposed in the first channel 1 through the first through opening 51. The other end of the salt bridge 3 and the second electrode 42 are exposed in the second channel 2 through the second through opening 52.

In some embodiments, the second plate 5 is configured to cover at least an area of the groove 43 of the first plate 4 to form the third channel for accommodating the salt bridge 3. An end of the third channel leads to the first channel 1, and the other end of the third channel leads to the second channel 2. The salt bridge 3 is capable of expanding to block the third channel after contacting a liquid.

In some embodiments, the sample test card 9 may also include a first housing 6. The first housing 6 and the first plate 4 clamp the second plate 5 together, forming the first channel 1 at a position corresponding to the first through opening 51 and the second channel 2 at a position corresponding to the second through opening 52. In this way, the first housing 6, the first plate 4, and the second plate 5 are configured to form the first channel 1 and the second channel 2 that are communicated with each other through the third channel.

In some embodiments, the groove 43 and the salt bridge 3 do not have to be arranged on the surface of the first plate 4. The groove 43 may be defined on a surface of a side of at least one of the first plate 4, the second plate 5, and the first housing 6 facing the first channel 1 and the second channel 2, as long as the liquid and the electrode in the first channel 1 may be conductive with the liquid and the electrode in the second channel 2 via the salt bridge 3.

In some embodiments, an inner wall of a partition portion 55 at a side of the second through opening 52 near the first through opening 51 forms a first depression 53. A portion of the salt bridge 3 may be exposed in the second channel 2 through the first depression 53. Due to certain limitations in the conductivity of the salt bridge 3 in a solid state, in order to minimize an impedance caused by the salt bridge 3 as much as possible, a length of the salt bridge 3 between the first channel 1 and the second channel 2 may be reduced as much as possible. By defining the first depression 53 as shown in FIG. 3, the second channel 2 may be closer to the first channel 1, and the portion of the salt bridge 3 exposed in the second channel 2 may be larger, which facilitates reducing the impedance and improving the accuracy of measuring the electrochemical parameter.

In some embodiments, in order to ensure sealing performance of the first channel 1 and the second channel 2, the second plate 5 may be made of a material with a certain elasticity and capable of undergoing a certain deformation, such as rubber. In this way, the first plate 4, the second plate 5, and the first housing 6 may achieve an interference fit to realize effects of preventing liquid leakage and isolating the first channel 1 from the second channel 2.

In some embodiments, since the second plate 5 may undergo a certain deformation, when a distance between the first through opening 51 and the second through opening 52 is too small and a width of the partition portion 55 between the first through opening 51 and the second through opening 52 is too small, the partition portion 55 is prone to excessive deformation due to insufficient structural strength under the influences of a liquid pressure or an impact force when the liquid is introduced into the first channel 1 or the second channel 2, resulting in the liquid leakages from the first channel 1 or the second channel 2 near the partition portion 55. Therefore, besides reducing the length of the salt bridge 3 between the first channel 1 and the second channel 2, it is necessary to provide sufficient supporting force for the first depression 53. For example, a shape of the first depression 53 may be set as arc-shaped, so as to ensure the stability of the structure by reducing the liquid pressure on the first depression 53 while keeping other parts of the partition portion 55 with sufficient width.

In some embodiments, due to the first depression 53, the liquid filled into the second channel 2 may form bubbles when flowing through the first depression 53, which may affect the electrical properties of the salt bridge 3. Therefore, an adsorbing element 56 may be arranged at the first depression 53 adjacent to the salt bridge 3. In this way, when the liquid in the second channel 2 flows through the first depression 53, the adsorbing element 56 is configured to adsorb the liquid in the second channel 2 to the salt bridge 3 or adsorb the liquid in the second channel 2 near the salt bridge 3, thereby discharging the bubbles near the salt bridge 3 and improving the accuracy of the test.

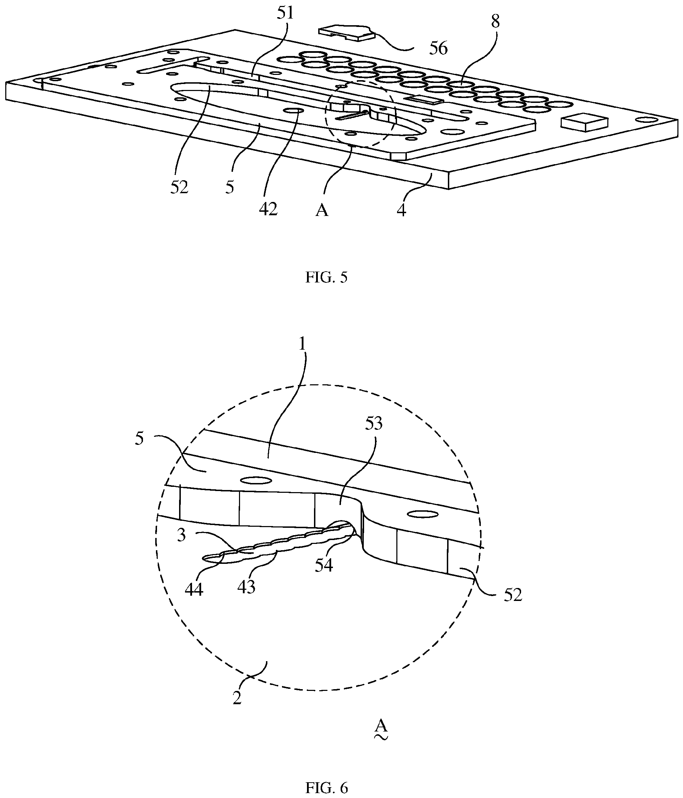

As shown in Fig. 5 and Fig. 6, FIG. 5 is a schematic assembled structural view of the first plate and the second plate shown in FIG. 1 from another view according to some embodiments of the present disclosure, and FIG. 6 is a schematic enlarged structural view of a region A shown in FIG. 5 according to some embodiments of the present disclosure.

The salt bridge 3 made of hydrogel material may expand after absorbing liquid, causing uncontrollable deformation of the salt bridge 3 that affects the conduction of the circuit. An arched space may be defined on a surface of a side of the second plate 5 facing the salt bridge 3 that covers partial surface of the salt bridge 3, so as to avoid the performance of the salt bridge 3 being affected by extrusion and deformation after expanding. As shown in FIG. 6, a first arched groove 54 is defined at a position of a surface of a side of the second plate 5 close to the salt bridge 3 and opposite to the groove 43. The salt bridge 3 is capable of expanding to the second plate 5 after absorbing a liquid, thereby filling the first arched groove 54.

In some embodiments, a depressed depth of the first arched groove 54 relative to a surface of a side of the second plate 5 towards the salt bridge 3 is related to the ability of the hydrogel of the salt bridge 3 to absorb liquid and a volume of the hydrogel in the groove 43. The first arched groove 54 is defined to be at least partially filled with the hydrogel after absorbing liquid and expanding, thereby ensuring the performance of the salt bridge 3 and avoiding mutual doping of the liquids in the first channel 1 and the second channel 2.

In some embodiments, considering that it is not convenient to process structures with overly small sizes and extremely high precision in mechanical processing, a volume of an arch space may be defined to be slightly larger than a volume of the salt bridge 3 after expanding. In order to offset an error caused by mechanical precision, an amount of the salt bridge 3 may be appropriately increased, so that the salt bridge 3 may effectively block the third channel after expanding and may not undergo severe deformation either.

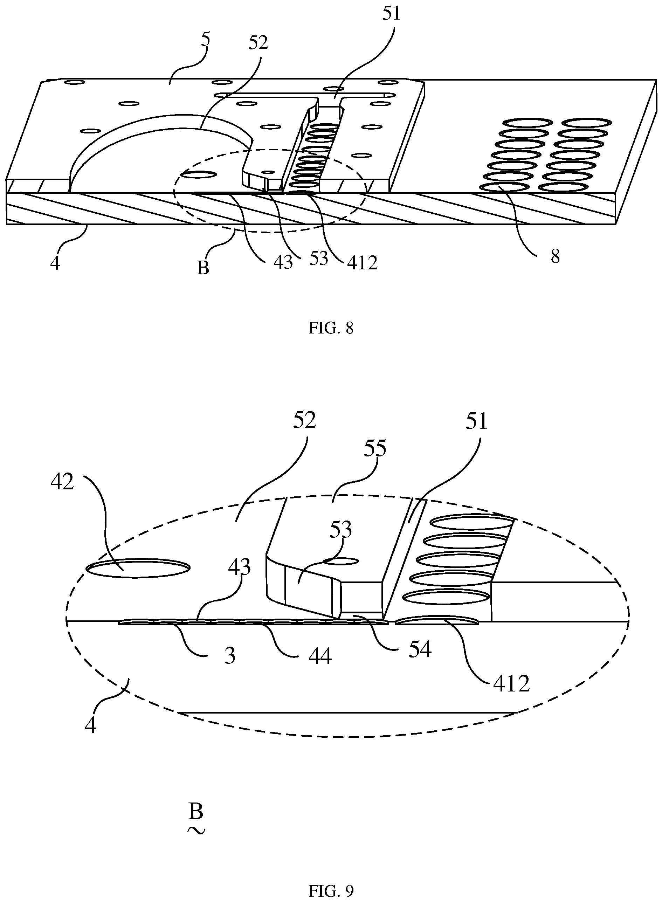

As shown in FIG. 8 and FIG. 9, FIG. 8 is a schematic cross-sectional view of FIG. 3 along a C-C direction according to some embodiments of the present disclosure, and FIG. 9 is a schematic enlarged structural view of a region B shown in FIG. 8 according to some embodiments of the present disclosure. The first arched groove 54 provided in the embodiments penetrates from a side of the first depression 53 to a side of the partition portion 55 close to the first through opening 51, so that a portion of the salt bridge 3 covered by or located in a projection of the first arched groove 54 projected on the first plate 4 may protrude from the groove 43 and may be completely filled in the first arched groove 54 after expanding.

In some embodiments, since the salt bridge 3 is the hydrogel containing the electrolyte, when the salt bridge 3 is filled into the groove 43, the salt bridge 3 may be filled by means of droplet-coating. In order to match a shape of the hydrogel that is dripped in a droplet shape and realize a better filling effect, as shown in FIG. 6, the groove 43 may be defined by a plurality of pits 44 that are partially overlapped. Each of the pits 44 is circular, quasi-circular, elliptical, quasi-elliptical, or polygonal in shape. The salt bridge 3 is filled in each of the pits 44 that is circular, quasi-circular, elliptical, quasi-elliptical, or polygonal in shape, thereby achieving a better filling effect of the salt bridge 3.

In some embodiments, the pits 44 may be arranged to partially overlapped one by one along a direction parallel to a connecting line of the first electrode 41 and the second electrode 42, and be communicated one by one in series to form the groove 43. In this way, the first electrode 41 may be better conducted to the second electrode 42. In addition, the material cost may be saved, and a space required for accommodating the salt bridge 3 is reduced.

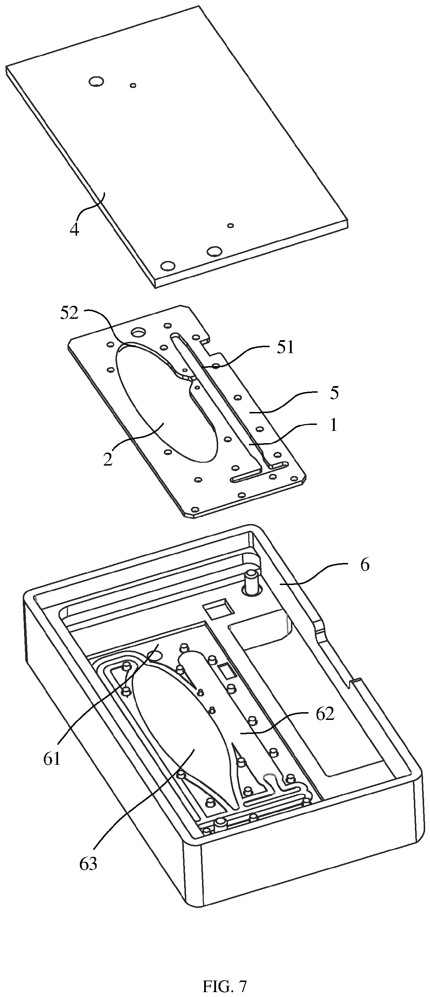

As shown in FIG. 7, FIG. 7 is a schematic structural view of the sample test card shown in FIG. 1 from another view according to some embodiments of the present disclosure. In order to achieve better measurement effect, certain spaces need to be designed in the first channel 1 and the second channel 2 of the sample test card to accommodate sufficient amount of liquids. Therefore, the first housing 6 may define a chamber 61.

A chamber wall of the chamber 61 may define a first fluid groove 62 corresponding to the first channel 1 and a second fluid groove 63 corresponding to the second channel 2. The plate is accommodated in the chamber 61. The first through opening 51 is communicated with a portion of the first fluid groove 62. The second through opening 52 is communicated with a portion of the second fluid groove 63. The plate seals another portion of the first fluid groove 62 and another portion of the second fluid groove 63. In this way, the first channel 1 and the second channel 2 are connected with each other only via the salt bridge 3.

Some embodiments of the present disclosure also provide a sample test card including a sample chamber 1b, a reference chamber 2b, a first electrode 41 located in the sample chamber 1b, and a second electrode 42 located in the reference chamber 2b. The first electrode 41 is configured to obtain an electrochemical parameter of a sample. The second electrode 42 is configured to obtain an electrochemical parameter of a reference solution. A third channel is defined between the sample chamber 1b and the reference chamber 2b. The third channel is configured to accommodate an electrolyte filler.

In some embodiments, the electrolyte filler may be in various forms of salt bridge.

In some embodiments, the sample and other solutions may be placed into the sample test card 9 by pre-storage or other means. Therefore, there is no need to define the liquid inlet 46a and the liquid outlet 46b in the sample test card 9, which may cause liquid leakage.

The embodiments mentioned above are only implementations of the present disclosure and do not limit the patent scope of the present disclosure. Any equivalent structures or equivalent process transformations made by using the contents of the description and the accompanying drawings of the present disclosure, or directly or indirectly applied in other related technical fields, are likewise included within the scope of patent protection of the present disclosure.

## Claims

1. A sample test card, comprising:
a first channel;
a second channel;
a first electrode, arranged in the first channel and configured to obtain an electrochemical parameter of a sample in the first channel;
a second electrode, arranged in the second channel and configured to obtain an electrochemical parameter of a reference solution in the second channel; and
a salt bridge, wherein an end of the salt bridge is exposed in the first channel, and the other end of the salt bridge is exposed in the second channel.

2. The sample test card according to claim 1, wherein
a surface area of a portion of the salt bridge exposed in the first channel is smaller than a surface area of another portion of the salt bridge exposed in the second channel; or
a length of the portion of the salt bridge exposed in the first channel is smaller than a length of the another portion of the salt bridge exposed in the second channel.

3. The sample test card according to claim 1, further comprising:
a third channel, configured to accommodate the salt bridge, wherein an end of the third channel is communicated with the first channel, the other end of the third channel is communicated with the second channel, and the salt bridge is capable of expanding to block the third channel after contacting a liquid.

4. The sample test card according to claim 1, further comprising:
a first plate, wherein the first electrode and the second electrode are arranged on the first plate, the end of the salt bridge is close to the first electrode, and the other end of the salt bridge is close to the second electrode;
a second plate, defining a first through opening and a second through opening, wherein the second plate is arranged on a side of the first plate arranged with the first electrode, the end of the salt bridge and the first electrode are exposed from the first through opening, and the other end of the salt bridge and the second electrode are exposed from the second through opening; and
a first housing, wherein the first housing and the first plate cooperatively clamp the second plate to form the first channel corresponding to the first through opening and the second channel corresponding to the second through opening;
wherein at least one of the first plate, the second plate, and the first housing defines a groove, and the groove is configured to accommodate the salt bridge.

5. The sample test card according to claim 4, wherein the groove is defined on a surface of a side of the first plate facing the first channel, the second plate is configured to cover at least an area of the groove of the first plate to form a third channel configured to accommodate the salt bridge, an end of the third channel leads to the first channel, the other end of the third channel leads to the second channel, and the salt bridge is capable of expanding to block the third channel after contacting a liquid.

6. The sample test card according to claim 5, wherein an inner wall of the second through opening close to the first through opening forms a first depression, and a portion of the groove is exposed in the second channel through the first depression.

7. The sample test card according to claim 4, wherein the groove is defined on a surface of a side of the first plate arranged with the first electrode; a surface of a side of the second plate close to the salt bridge and opposite to the groove defines a first arched groove, and the salt bridge is capable of expanding to the second plate after absorbing a liquid such that the first arched groove is filled with the expanded salt bridge.

8. The sample test card according to claim 4, wherein the groove is defined by a plurality of pits that are partially overlapped, each of the pits is circular, quasi-circular, elliptical, quasi-elliptic, or polygonal in shape, and the salt bridge is filled in each of the pits that is circular, quasi-circular, elliptical, quasi-elliptic, or polygonal in shape.

9. The sample test card according to claim 8, wherein the pits are arranged to be partially overlapped one by one along a direction parallel to a connecting line of the first electrode and the second electrode, and are communicated in series to form the groove.

10. The sample test card according to claim 4, wherein the first plate comprises a circuit board, and the circuit board is arranged on a side of the first plate close to the first channel and the second channel; and
wherein the salt bridge, the first electrode, and the second electrode are arranged on a surface of a side of the circuit board facing the first channel.

11. The sample test card according to claim 4, wherein the first plate defines a liquid inlet and a liquid outlet, and the sample test card further comprises:
a first valve, a second valve, and a third valve;
wherein the liquid inlet is communicated with an end of the first channel, the other end of the first channel comprises a first branch and a second branch, the first branch is communicated with the liquid outlet through the first valve, the second branch is communicated with an end of the second channel through the second valve, and the other end of the second channel is communicated with the liquid outlet through the third valve.

12. The sample test card according to claim 1, wherein a distance between the salt bridge and the first electrode is smaller than a distance between the salt bridge and the second electrode.

13. The sample test card according to claim 1, wherein an impedance of the salt bridge ranges from 1 kΩ to 1000 kΩ.

14. The sample test card according to claim 1, wherein a material of the salt bridge is hydrogel, agar and/or silica gel.

15. A sample test card, comprising:
a sample chamber;
a reference solution chamber;
a first electrode, arranged in the sample chamber and configured to obtain an electrochemical parameter of a sample; and
a second electrode, arranged in the reference solution chamber and configured to obtain an electrochemical parameter of a reference solution; and
wherein a third channel is defined between the sample chamber and the reference solution chamber, and the third channel is configured to accommodate an electrolyte filler.
